# EUROPEAN PATENT SPECIFICATION

(11) **EP 4 346 969 B1**
(45) Date of publication and mention of the grant of the patent: **11.02.2026**
(21) Application number: 22740558.6
(22) Date of filing: 26.05.2022
(51) Int. Cl.: A61M 16/16

(54) **RESPIRATORY THERAPY SYSTEM WITH HUMIDITY CONTROL USING WATER FLOW**
ATEMTHERAPIESYSTEM MIT FEUCHTIGKEITSSTEUERUNG MITTELS WASSERFLUSS
SYSTÈME DE THÉRAPIE RESPIRATOIRE AVEC CONTRÔLE DE L'HUMIDITÉ AU MOYEN D'UN ÉCOULEMENT D'EAU

(30) Priority: 28.05.2021 US 202163194595 P
(43) Date of publication of application: 10.04.2024
(73) Proprietor: Vapotherm, Inc., Exeter, NH 03833 (US)
(72) Inventor: WINSTON, David, Exeter, NH 03833 (US); LEONARD, Scott, A., Exeter, NH 03833 (US); DAVIDSON, Marc, Exeter, NH 03833 (US)
(74) Representative: Larsen, Charles
(86) International application number: PCT/US2022/031170
(87) International publication number: WO 2022/251525

(56) References cited:
- EP-A1- 3 363 489
- WO-A1-2012/077052
- WO-A1-2012/080941
- WO-A1-2016/161092
- WO-A1-86/02276
- WO-A2-2008/030592
- US-B2- 10 864 346

## Description

### BACKGROUND

In conventional respiratory therapy systems, breathing gas is often humidified prior to delivery of the gas to the patient, in order to increase patient comfort and prevent drying out of the patient's airways, especially in the administration of high flow therapy (HFT) or high velocity therapy (HVT), such as high velocity nasal insufflation (HVNI). Accordingly, the humidity level of administered breathing gas, along with flowrate and temperature, is an important parameter in controlling these systems.

WO 2016/161092 A1 describes a system for humidifying a breathing gas. The system includes a source of pressurized breathing gas, an external vapor transfer unit, a first gas tube connecting the source of pressurized breathing gas to the gas inlet of the vapor transfer unit and having a first length, a liquid supply having a heater that heats liquid, a first liquid tube coupling the liquid supply to the liquid inlet of the vapor transfer unit, and a second gas tube having a second length and connecting the gas outlet of the vapor transfer unit to a patient interface. The first length is greater than the second length. The vapor transfer unit includes a gas passage, a liquid passage, and a vapor permeable membrane separating the gas passage and the liquid passage. WO 2008/030592 A2 describes a system and method for delivering medication to patients. The system includes an infuser including a membrane. The infuser entrains a fluid from a first side of a membrane and a medication into a gas on a second side of the membrane to form a medicated humidified gas. A delivery system is coupled to the infuser. The delivery system is configured to deliver the medicated humidified gas to the patient. The method includes entraining a fluid on a first side of a membrane and a medication into a gas on a second side of the membrane to form a medicated humidified gas and delivering the medicated humidified gas to the patient using a delivery system. WO 86/02276 A1 describes a method and an apparatus which are useful for respiratory tract therapy. The method comprises contact of mucous membranes with vapor-phase water in the substantial absence of nucleating water-vapor condensation particulate, and includes the use of membrane means disposed within a membrane container having a gas inlet, a vapor-gas outlet, a liquid inlet and liquid outlet, a heated delivery tube, unheated nasal cannula, for the delivery of a nasal cannula vapor/gas stream by the use of exit use ports to a nasal passageway at dew point temperatures equal to or less than dry bulb temperatures and heating of a delivery tube vapor-gas stream to a temperature sufficiently greater than the cannula nare dew point use temperature. WO 2012/080941 A1 describes systems for humidifying gas delivered to a patient. A humidifier unit is provided located proximal to a patient interface of a patient circuit. The humidifier unit includes a chamber that receives water from a water source, a heat source disposed within the chamber, a water flow sensor, an output temperature sensor, an output humidity sensor, and/or other sensors. A controller receives flow data relating to water being provided to the chamber and temperature data relating to the temperature of the humidified gas, humidity, and/or other data. The controller further adjusts a flow of water being provided to the chamber based on the flow data and/or a heat output of the heat source based on the received data. WO 2012/077052 A1 describes a humidifier for use in a patient ventilation circuit. The humidifier comprises: an inlet configured to receive a gas from a ventilator; an outlet configured to provide a humidified gas to a patient; a chamber disposed between the inlet and the outlet; a reservoir substantially surrounding the chamber and configured to hold a liquid; and a hydrophobic membrane disposed between the reservoir and the chamber. Vapor of the liquid passes through the hydrophobic membrane and into the chamber. US 10,864,346 B2 describes a medical humidifier for humidification of air to be delivered to a patient's airways. The humidifier may include a humidification chamber, a reservoir and a water delivery mechanism. The humidification chamber may include a water retention feature such as a wick, a heating element for heating the humidification chamber, and an air flow baffle configured to promote humidification. The humidifier may be further configured to execute one or more algorithms, for example to determine a condition of the humidifier and/or to mitigate any detected faults. In some forms, the humidifier may also comprise algorithms for controlling one or more components of the humidifier. EP 3363489 A1 describes a respiratory assistance device that includes a gas supply source, an inspiratory pipe, and a humidifier. A gas is supplied to a mask through the gas supply source and the inspiratory pipe. The humidifier includes a liquid supply unit, a humidifying member, a holder unit, a heating unit, and the like. The liquid supply unit supplies a liquid to the humidifying member. The humidifying member has an aspect that makes one round of the inner periphery of the inspiratory pipe, and vaporizes the liquid supplied from the liquid supply unit. The holder unit holds the humidifying member such that the humidifying member is disposed to make one round of the inner periphery of the inspiratory pipe in a continuous or discontinuous manner. The heating unit heats the inside of the inspiratory pipe.

### SUMMARY OF THE INVENTION

The present invention provides a respiratory therapy system as defined in claim 1. Further preferred embodiments of the present invention are defined in the dependent claims.

### SUMMARY OF THE DISCLOSURE

The systems, devices, and methods described herein relate to control of humidification of breathing gas provided by a respiratory therapy system. The methods described herein allow for control of breathing gas humidity independent of gas temperature and gas flowrate, thereby allowing a user or physician to independently set and control each of the temperature, flowrate, and humidity.

**In** one aspect, there is provided a respiratory therapy system according to claim 1. The system comprises (i) a gas supply module configured to supply a flow of breathing gas; (ii) a liquid supply module configured to supply a flow of liquid; and (iii) a vapor transfer module configured to receive the flows of gas and liquid, humidify the gas, and output heated and humidified gas. The system is configured to (a) supply the flow of breathing gas from the gas supply module to the vapor transfer module; (b) supply the flow of liquid from the liquid supply module to the vapor transfer module at a first liquid flowrate; (c) output the heated and humidified gas from the vapor transfer module at a first gas temperature, a first gas flowrate, and a first humidity; (d) adjust the first liquid flowrate to a second liquid flowrate; and (e) output the heated and humidified gas from the vapor transfer module at about the first gas temperature, about the first gas flowrate, and a second humidity. A difference between the first humidity and second humidity is inversely proportional to the adjustment of the first liquid flowrate to the second liquid flowrate.

### BRIEF DESCRIPTION OF THE DRAWINGS

The foregoing and other objects and advantages will be apparent upon consideration of the following detailed description, taken in conjunction with the accompanying drawings, in which like reference characters refer to like parts throughout, and in which:
FIG. 1A shows a chart of gas and water temperatures along a vapor transfer cartridge and delivery tube, according to an illustrative implementation;
FIG. 1B shows an adjusted chart of gas and water temperatures along a vapor transfer cartridge and delivery tube, according to an illustrative implementation;
FIG. 2 shows a respiratory therapy system, according to an illustrative implementation;
FIG. 3 shows a liquid supply module, according to an illustrative implementation; and
FIGs. 4A-4F show various views and cross-sections of a vapor transfer module and liquid supply module, according to an illustrative implementation.

### DETAILED DESCRIPTION

To provide an overall understanding of the systems, method, and devices described herein, certain illustrative embodiments will be described. Although the embodiments and features described herein are specifically described for use in connection with specific respiratory therapy systems, it will be understood that all the components and other features outlined below may be combined with one another in any suitable manner and may be adapted and applied to other types of respiratory therapy systems. The term "about" may be understood to mean within 20% or less of a value; for example, "about 40 L/min" can be understood as 32-48 L/min.

The systems, devices, and methods described herein relate to techniques for adjusting humidity of a breathing gas supplied to a patient from a respiratory therapy system. Some systems humidify breathing gas by flowing a liquid (*e.g.*, water) past the gas in a vapor transfer device (*e.g.,* a vapor transfer cartridge containing membrane(s), a convective flow humidifier). Such systems can be limited as to how many (and which) of the gas parameters (flowrate, temperature, and humidity) can be controlled independently of each other and with respect to parameters of liquid used for humidification, *i.e.,* the liquid flowrate and liquid temperature. For example, a vapor transfer cartridge typically has a characteristic curve, which determines, for a given liquid temperature, liquid flowrate, and gas flowrate, what the absolute humidity of the output gas will be.

The variables (gas and liquid parameters) are further constrained when the same liquid that is used for humidification is also used for insulation of the breathing gas, because the liquid temperature of humidification is directly tied to the liquid temperature of insulation, which affects the gas temperature. At lower flowrates, the humidity will be higher and closer to the saturation level for the given gas temperature, which can lead to rain-out of the humidity into the gas flow path. At high flowrates, the humidity can be too low, which could be uncomfortable or harmful to the patient. Thus, the system is forced to compromise between low and high flowrates to accommodate the humidification constraints, or a different humidification technique must be used for different flowrate ranges, which narrows the range available to the physician when administering therapy with a given system and humidification technique. Accordingly, a method for independently controlling humidity would allow for a better range of therapy control on a given device.

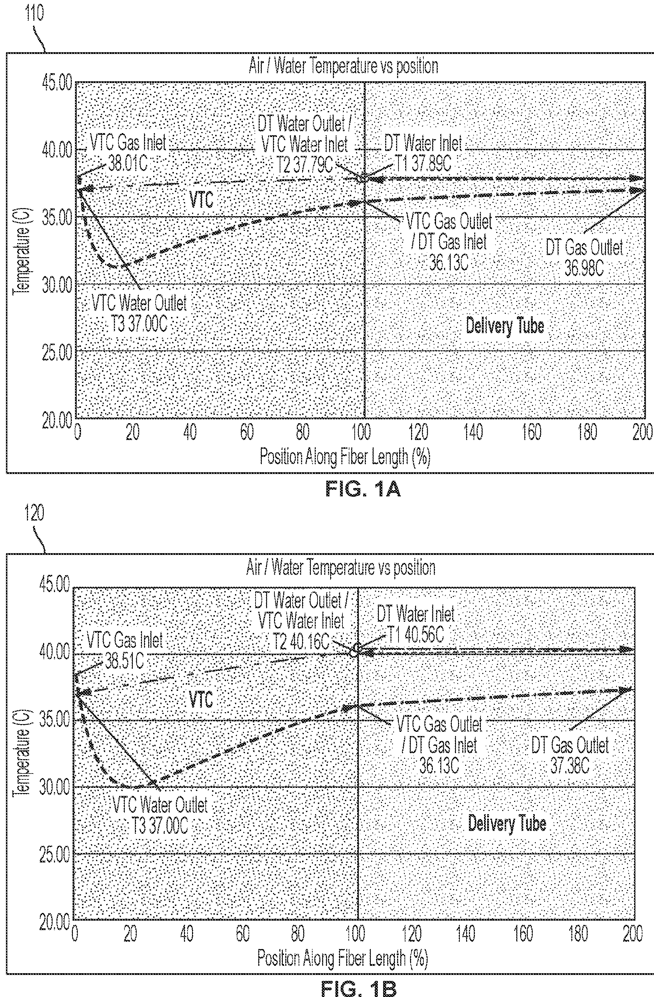

FIG. 1A shows a chart of gas and water temperatures for a system with a vapor transfer cartridge (VTC) and a water jacketed delivery tube. The temperatures charted in FIG. 1A are based on a gas flowrate setpoint of 45 L/min (body temperature and pressure, saturated (BTPS)) and a water flowrate setpoint of 1 L/min. BTPS is a standard flow, similar to standard liters per minute (SLPM). SLPM is a mass flowrate measure that corresponds to a volume flow at sea level, 20 °C, and 0% humidity. So 40 SLPM at high pressure may have a volumetric flowrate that is less than 40 L/min. BTSP references conditions inside the human body, so 45 L/min BTPS is the flow that will result in 45 L/min when the gas is heated to 37 °C, at the pressure of the patient's environment, and completely saturated with vapor. The left portion (0-100) of the chart shows the gas and water temperatures in the VTC, while the right portion (100-200) shows the gas and water temperatures in the delivery tube. In the delivery tube, the same water that is used to humidify breathing gas is circulated through the delivery tube jacket to prevent condensation from the humidified gas before delivery to the patient. In this system, the flow path of gas is (in order) the VTC gas inlet, the VTC gas outlet, the delivery tube gas inlet, and the delivery tube gas outlet. The flow path of water is (in order) the delivery tube water inlet, the delivery tube water outlet, the VTC water inlet, and the VTC water outlet. The VTC is arranged with counter current flow, such that the water and gas flow through the VTC in opposite directions, with the gas inlet and water outlet are disposed at the same end of the VTC, and the gas outlet and water inlet are disposed at the same (opposite) end of the VTC. The water flows through the delivery tube jacket in an outward direction (from the gas inlet towards the gas outlet), then returns back towards the VTC.

In this example, the water temperature drops from 37.9 °C (at the delivery tube water inlet) to 37.8 °C (at the delivery tube water outlet). The water then enters the VTC and flows through the VTC from the water inlet to the water outlet, where the temperature has dropped to 37 °C. The gas enters the VTC gas inlet at 38 °C and flows to the VTC gas outlet while heat and moisture are exchanged with the water flowing in the opposite direction. At the VTC gas outlet, the gas reaches 30.2 mg/L humidity. Along the delivery tube heat is exchanged with the water in the jacket, bringing the gas to a temperature of 37 °C.

Provided herein is a method of adjusting the humidity of the gas by varying the liquid flow rate. Counterintuitively, lowering the water flowrate can be used to increase the humidity of the output gas for the same gas temperature and flowrate, when the temperature of the liquid exiting the humidifier is held constant. Lowering the liquid flowrate increases the liquid temperature gradient within the humidifier. If the liquid outlet temperature is held constant, then the liquid inlet temperature must be higher to account for the lower flowrate. The higher liquid inlet temperature increases the vapor pressure gradient between the liquid and the gas. Since the humidifier is in a counter-current arrangement, the higher temperature liquid is exchanging vapor with the gas that is about to exit the humidifier. The evaporation increases the water content of the gas, while evaporative cooling reduces how much the gas temperature rises. The result is that the gas output of the humidifier has a similar temperature when compared to a higher liquid flowrate, but has a greater humidity.

FIG. 1B shows a chart of gas and water temperatures for the same example system as used for FIG. 1A, with the same gas flowrate and temperature setpoints, but the liquid flowrate is reduced to 0.5 L/min. The VTC water outlet is held at a constant 37 °C, and the gas outlet temperature has only risen by 0.4 °C. The VTC water inlet, however, is 2.4 °C warmer, which increases the humidity in the gas from 30.2 mg/L to 35.0 mg/L.

In some systems or therapies, the liquid flowrate may be inversely proportional to the gas flowrate. This will reduce humidity at lower gas flowrates to prevent rainout (condensation of water vapor in the delivery tube), and increase humidity at higher flowrates to improve humidification.

The foregoing is merely illustrative of the principles of the disclosure, and the apparatuses can be practiced by other than the described embodiments, which are presented for purposes of illustration and not of limitation. It is to be understood that the apparatuses disclosed herein, while shown for use in specific respiratory therapy systems, may be applied to other respiratory therapy systems requiring humidification.

The system includes: (i) a gas supply module configured to supply a flow of breathing gas; (ii) a liquid supply module configured to supply a flow of liquid; and (iii) a vapor transfer module configured to receive the flows of gas and liquid, humidify the gas, and output heated and humidified gas.

The liquid supply module includes a heater configured to heat the liquid (e.g., water), a pump configured to circulate the liquid, and, optionally, a reservoir configured to hold liquid and having an outlet port and a return port. Where provided, the return port is in fluid communication with the vapor transfer module and configured to convey liquid from the vapor transfer module to the reservoir.

The vapor transfer module includes a vapor transfer cartridge and a delivery tube. The vapor transfer cartridge has a gas inlet, a gas outlet, a liquid inlet, and a liquid outlet. The gas inlet receives the flow of breathing gas from the gas supply module, and the gas outlet outputs the heated and humidified breathing gas.

The delivery tube may have a gas lumen and a liquid lumen at least partially surrounding the gas lumen. The gas lumen is in fluid communication with the gas outlet of the vapor transfer cartridge so as to receive the heated and humidified gas from the gas outlet of the vapor transfer cartridge. The delivery tube may have a first end and a second end, where the gas lumen receives the heated and humidified gas at the first end and conveys the heated and humidified gas to the second end. The liquid lumen may have a first channel and a second channel. The first channel is configured to convey liquid from the first end to the second end, and the second channel is in fluid communication with the first channel at the second end and configured to convey the liquid from the second end to the first end. In some implementations, the second channel at the first end is in fluid communication with the liquid inlet of the vapor transfer cartridge, and the liquid inlet receives liquid from the second channel. In some implementations, the delivery tube further includes a first temperature sensor disposed at an inlet of the first channel and a second temperature sensor disposed at an outlet of the second channel. The first temperature sensor and second temperature sensor each measure a temperature, and a temperature difference between the measured temperatures can be less than 5%. In some implementations, a temperature difference between the measured temperatures is less than 20%, less than, 15%, less than 10 %, or less than 5% of the first gas temperature. The temperature difference can be less than 1 °C. In some implementations, the temperature difference is less than 5 °C, 4°C, 3 °C, 2°C, or 1 °C.

In some implementations, the vapor transfer module and the liquid supply module define a liquid circulation loop, whereby liquid flows from the reservoir, to the heater, to the liquid lumen of the delivery tube, to the liquid inlet of the vapor transfer cartridge, through the vapor transfer cartridge, and to the reservoir via the return port. The liquid outlet of the vapor transfer cartridge is configured to convey liquid from the vapor transfer cartridge to the reservoir via the return port. In some implementations, the gas inlet and liquid outlet are disposed at a first end of the vapor transfer cartridge, and the gas outlet and liquid inlet are disposed at a second end of the vapor transfer cartridge, such that the liquid and gas flow through the vapor transfer cartridge in opposite directions. In some implementations, a liquid temperature at the liquid inlet is held constant before and after a temperature adjusting step as described above.

**In** some implementations, the system includes a nasal cannula coupled to the delivery tube and configured to convey the heated and humidified gas to the nares of a patient. In some implementations, the vapor transfer cartridge includes a plurality of hollow fibers. In some implementations, the vapor transfer cartridge includes a pleated membrane or a flat sheet membrane. In some implementations, the vapor transfer cartridge includes a heated wick. In some implementations, the gas supply module includes a blower configured to output the flow of breathing gas. In some implementations, the system includes a controller operatively coupled to the gas supply module and the liquid supply module and configured to adjust at least one of a gas temperature setpoint, a gas flowrate setpoint, a liquid flowrate setpoint, or a liquid temperature setpoint.

In some implementations, the second liquid flowrate is less than the first liquid flowrate, and the second humidity is greater than the first humidity. In some implementations, the second liquid flowrate is greater than the first liquid flowrate, and the second humidity is less than the first humidity. The first gas temperature can be 30 to 40 °C. In some implementations, the first gas temperature is 25 to 45 °C, or 30 to 40 °C, or 32 to 38 °C. The first gas flowrate can be 5 to 45 L/min. In some implementations, the first gas flowrate is 3 to 60 L/min, 4 to 50 L/Min, 5 to 45 L/min, 10 to 40 L/min, or 15 to 35 L/min. At least one of the first humidity or the second humidity can be 25-45 mg/L. In some implementations, at least one of the first humidity or the second humidity is 10-60 mg/L, 15-55 mg/L, 20-50 mg/mL, or 25-45 mg/L.

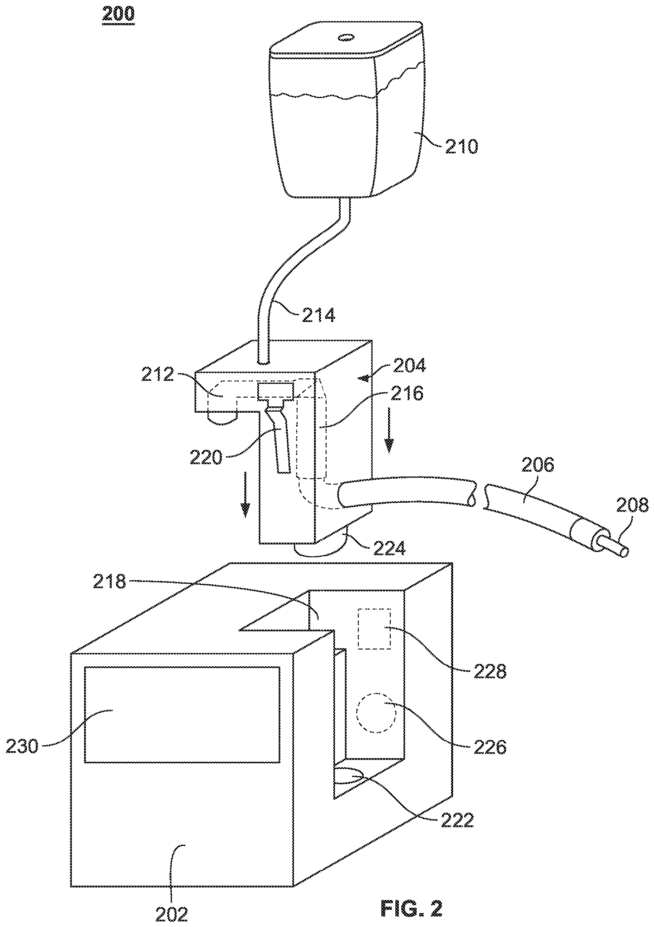

FIG. 2 shows an example respiratory therapy system 200 on which this method of humidity control may be implemented. System 200 comprises a base unit 202, an auxiliary unit 204, and a delivery tube 206. Base unit 202 contains a controller (not shown), and base unit 202 is configured to direct a flow of breathing gas into auxiliary unit 204 when auxiliary unit 204 is operatively coupled to base unit 202. The controller of base unit 202 can be a computing device including digital electronic circuitry, or in computer software, firmware, or hardware, including a processor and a computer program as described in this specification. Auxiliary unit 204 directs the breathing gas through gas path 212 to vapor transfer cartridge (VTC) 216 disposed within auxiliary unit 204. Auxiliary unit 204 is configured to receive a liquid (*e.g.,* water) from external reservoir 210 through external liquid tube 214. Breathing gas flows from an outlet of VTC 216 into delivery tube 206 for delivery of the breathing gas to the patient. Delivery tube 206 includes a patient connector 208. For the operative coupling of auxiliary unit 204 to base unit 202, base unit 202 includes a recess 218 sized to receive auxiliary unit 204, and auxiliary unit 204 includes a latch 220 for locking auxiliary unit 204 in recess 218. Auxiliary unit 204 includes a pump 224 configured to pump the liquid. Base unit 202 includes various couplings 222, 226, and 228 that are configured to interact with auxiliary unit 204 or components thereof. A display 230 is included on base unit 202.

Base unit 202 may contain a blower configured to produce a flow of breathing gas. In the following, system 200 is described using a blower as an example; however, it is to be understood that base unit 202 (and the other base units described herein) may alternatively be connected to an external gas source such as a wall air outlet, an air tank, or an external blower. Furthermore, a VTC 216 is implemented in system 200; however, it is to be understood that alternatives may be implemented in system 200 (and the other systems described herein), such as a heated wire or hot pot humidifier. The alternatives may operate by disposing water over a heating plate in a chamber and flowing breathing gas into the chamber (either over the water or through the water) to be humidified. A heated wire can be disposed in a conduit to further heat the humidified breathing gas before delivering the breathing gas to a patient.

System 200 may further include a nasal cannula (not shown) coupled to patient connector 208. Alternatively, patient connector 208 may be a nasal cannula or other gas delivery device. In either case, system 200 may be operated with system parameters for high velocity respiratory therapy (e.g., the parameters in Tables 1 and 2). The nasal cannula or patient connector may be sized accordingly, and/or the blower may be operated at certain breathing gas flowrates. By using a blower, system 200 may absolve a need for an external source of pressurized air or breathing gas such as a pressurized tank or a wall air outlet.

**Table 1: Exemplary combinations of nasal prong inner diameter and maximum flow setpoint configured to provide high velocity respiratory therapy on systems of the present disclosure.**

| **Nasal Prong Inner Diameter d (mm)** | **Maximum Flow Setpoint Q (L/min)** |
|---|---|
| 1.4 ≤ d < 1.8 | 9 ≤ Q < 20 |
| 1.8 ≤ d < 1.9 | 13 ≤ Q < 31 |
| 1.9 ≤ d < 3 | 21 ≤ Q < 60 |
| 3 ≤ d < 4 | 34 ≤ Q < 80 |

In some implementations, there are certain cannula sizes that can be used on the high velocity respiratory therapy systems discussed herein due to certain system conditions or properties of the cannula. For example, there may be allowable cannula pressure drops for a given nasal prong inner diameter and flow setpoint. Table 2 shows exemplary ranges of cannula pressure drops for certain pressure drops and flow setpoints.

**Table 2: Exemplary nasal cannula pressure drops that can allow high velocity respiratory therapy with the described system based on nasal prong inner diameter and flow setpoints.**

| **Nasal Prong Inner Diameter d (mm)** | **Cannula Pressure Drop (hPa)** | **Flow Setpoint (L/min)** |
|---|---|---|
| 1.1 ≤ d < 1.6 | 80 | 8 |
| 1.5 ≤ d < 2 | 100 | 20 |
| 1.9 ≤ d < 3.5 | 80 | 40 |

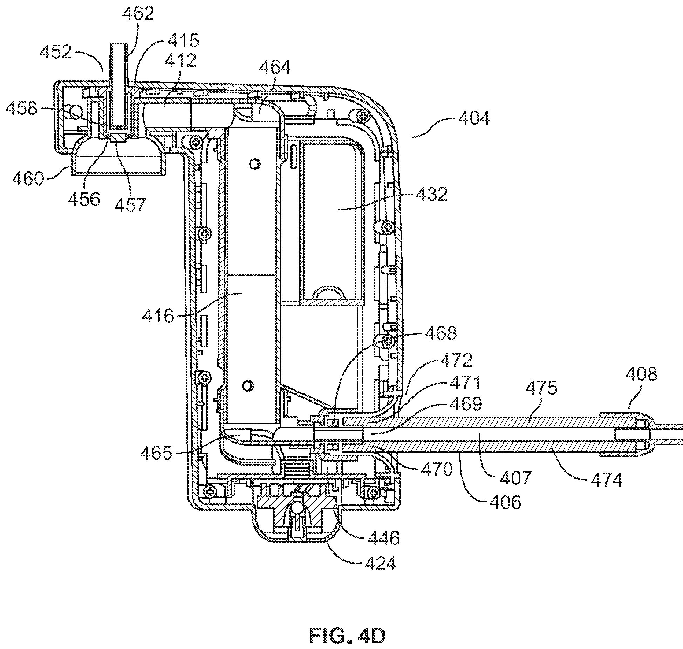

In some implementations, external reservoir 210 is a large container (>1 L, >2 L, >5 L, etc.) such as a bag or bottle, and the connecting tube 214 can be removably connected to auxiliary unit 204. Auxiliary unit 204 may be configured such that the liquid is fully enclosed in auxiliary unit 204 flowing from external reservoir 210. In this case, liquid is kept separate from base unit 202. This may prevent leaks and damage of components within base unit 202. In some implementations, gas path 212 is connected to the blower of base unit 202, but there is no other fluid communication between base unit 202 and auxiliary unit 204. Gas path 212 may be connected to base unit 202 by a valve, such as an occluder valve 452 of FIG.4D. Such valve may be used to control the flow rate of breathing gas entering gas path 212 of auxiliary unit 204. The valve may also interact with liquid flow to simultaneously control breathing gas flowrate and liquid flowrate. The valve may comprise components that are separately attached to base unit 202 and auxiliary unit 204, such that the valve is operable when auxiliary unit 204 is fully seated in recess 218 of base unit 202, and the controller of base unit 202 actuates the valve. A gas seal may be attached to auxiliary unit 204, in order to prevent gas leaks out of system 200 and to prevent liquid ingress into base unit 202 during operation. The gas seal may be a flexible portion that surrounds the valve, and the flexible portion is compressed to form a seal when auxiliary unit 204 is fully seated in recess 218 of base unit 202. The flexible portion of the gas seal, throughout this disclosure, may be a film, a gasket, a ring, or a collar, or other suitable mechanism.

While base unit 202 and auxiliary unit 204 are fluidically connected to at least allow breathing gas flow, it can be advantageous to otherwise minimize fluidic connections or contact between base unit 202 and auxiliary unit 204. For example, minimizing fluidic contact with base unit 202 may prevent damage to electronic components. As another example, previous systems have relied on conductive heating of liquid, which requires precise contact between two plates; thus, conductive heating is inefficient if precise contact cannot be established due to misalignment or deformation. Accordingly, couplings 222, 226, and 228 are configured to be contactless or non-contact couplings. Generally, this allows a sealed auxiliary unit without any openings and minimal requirements for tight tolerances. In some implementations, coupling 222 is a stator configured to magnetically couple to pump 224, which includes a rotor. Pump 224 may be shaped like a cup with a convex side protruding from auxiliary unit 204, and the stator may surround a cavity in recess 218, such that pump 224 sits within the cavity when auxiliary unit 204 is seated in recess 218. Pump 224 and coupling 222 may each be hermetically sealed, for example, to prevent fluid ingress into base unit 202. When auxiliary unit 204 is fully seated in base unit 202, the controller can operate the stator to magnetically generate rotation of the rotor of pump 224 to pump liquid through the auxiliary unit or through a jacket of delivery tube 206. As a result, the auxiliary unit 204 pumps liquid without an internalized power source or other electronic components to control the pump 224, and there is no need for liquid to flow through the base unit 202, which separately houses the controller and other components that may be susceptible to, e.g ., water damage. Pump designs compatible with the present disclosure are described in U.S. Patent Application Publication No. 2018/0104436 A1.

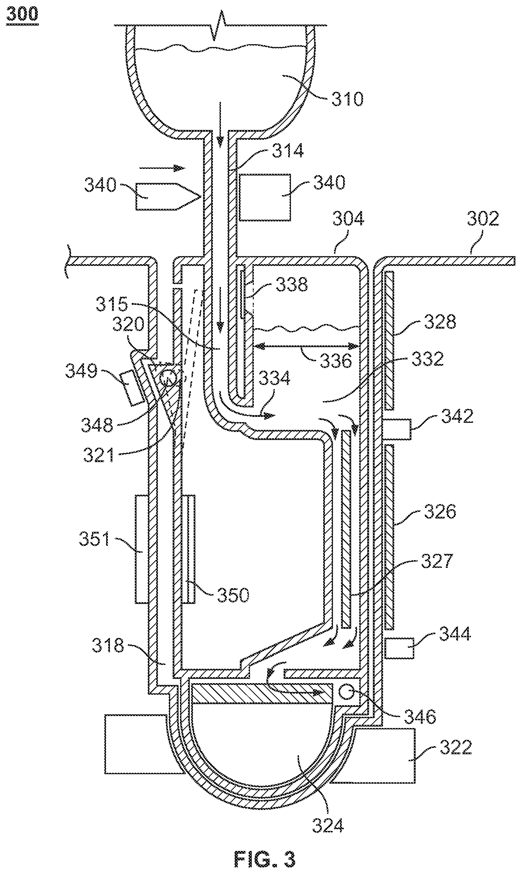

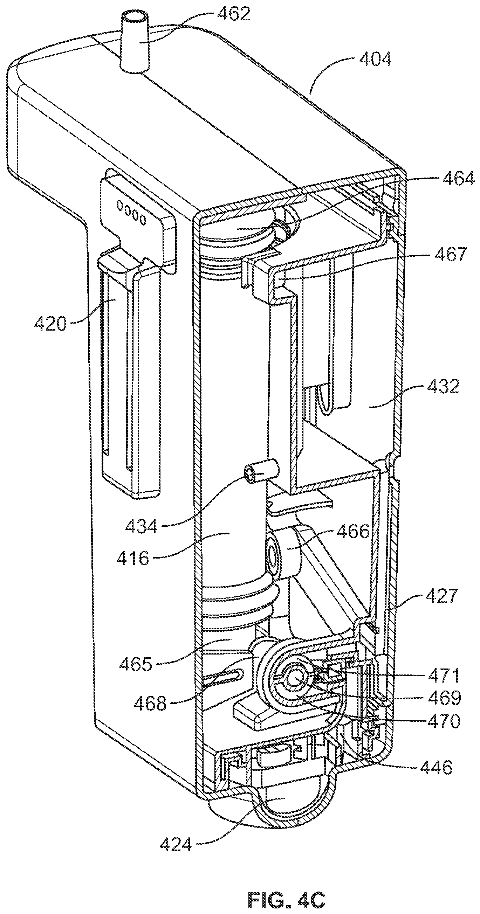

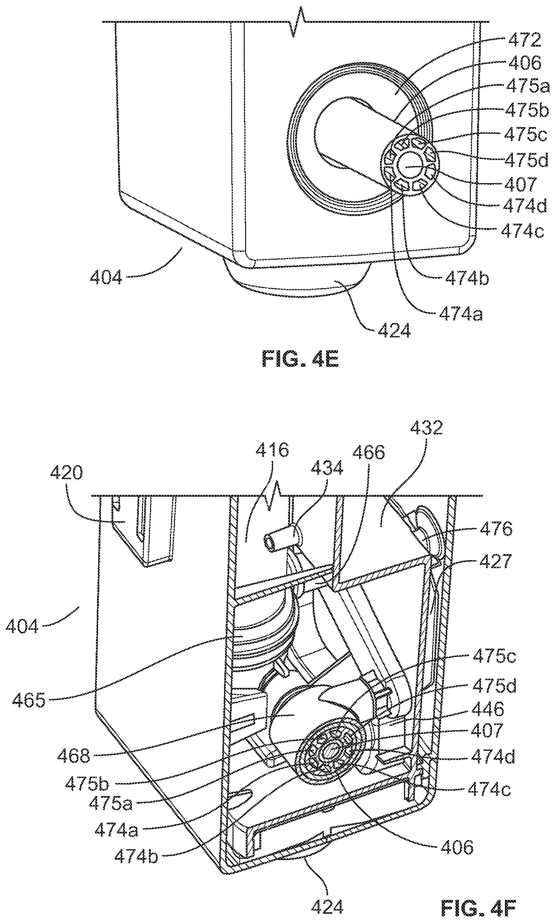

Similarly, coupling 226 may be a contactless or non-contact heating actuator, for example, heat actuator 326 of FIG. 3, used to convey energy to auxiliary unit 204 to heat the liquid. In some implementations, coupling 226 is a coil configured to heat a plate disposed in auxiliary unit 204. The plate may be, for example, the heating plates 327 or 427 of FIGs. 3, 4C, and 4F. The coil may heat the plate via induction. This may involve the coil inducing a current in the plate that is circular in shape and immersed in liquid in auxiliary unit 204. The induced current generates heat in the plate due to a resistance of the plate. Because the heat is generated in the plate that is surrounded by liquid, external surfaces of auxiliary unit 204 and base unit 202 that can be touched by a user/operator never exceed the liquid temperature. This configuration eliminates the need for precisely manufactured flat plates to get intimate contact between a heat source in base unit 202 and a conductive plate in auxiliary unit 204. Inductive heating improves efficiency of heating, because the generated heat is isolated to auxiliary unit 204 and does not heat the base unit 202. If base unit 202 were heated by excess heat, additional cooling would be required to remove the heat; however, additional cooling is unnecessary with the present inductive heating configuration. Improved efficiency also allows longer operation of system 200 if power is supplied by a battery (not shown) in situations where wall power is not available, e.g., during mobile use or power outages.

Heated liquid is used to heat, in turn, breathing gas. Optionally, delivery tube 206 may include a jacket configured to receive heated liquid and convey the heated liquid around an inner lumen that conveys the breathing gas. Heated liquid may travel along a full length of delivery tube 206 in a first direction towards patient connector 208, and then return in an opposite direction back to auxiliary unit 204. Heated liquid is conveyed into VTC 216 and may be vaporized into the breathing gas. For example, the liquid may be water used to humidify the breathing gas. Accordingly, the liquid may be heated to a target temperature, where the target temperature is a temperature at which the gas is to be delivered to the patient. The liquid may be circulated through auxiliary unit 204 and delivery tube 206 and back to the internal reservoir. In some implementations, the liquid travels a liquid path from the external reservoir to the internal reservoir (internal to auxiliary unit 204), then to a heating section (e.g, where the heating plate may be disposed), then through pump 224, then into the heating jacket of delivery tube 206, then into VTC 216, and then any remaining, un-vaporized liquid is returned to the internal reservoir. A similar configuration is described in further detail in relation to FIG. 3.

Similar to coupling 226, coupling 228 may be a contactless or non-contact level sensor, for example, level sensor 328 of FIG. 3, configured to detect a liquid level in auxiliary unit 204. For example, auxiliary unit 204 may contain an internal reservoir, such as the one described above and internal reservoirs 332 and 432 of FIGs. 3, 4C, 4D, and 4F. Liquid is stored in the internal reservoir for use, e.g, in VTC 216 and/or heating jacket of delivery tube 206. When the liquid is water that is vaporized into the breathing gas within VTC 216, it may desirable for the system or user to know the humidity of breathing gas provided to the patient. In some implementations, the internal reservoir has a constant diameter or cross-section (for example, cross-section 336 of internal reservoir 332 of FIG. 3), so that the volume of liquid is linearly proportional to the liquid level in the reservoir. In this implementation, level sensor 228 is configured to measure the liquid level when auxiliary unit 204 is fully seated in base unit 202. Level sensor 228 may be a capacitive sensing circuit positioned alongside the internal reservoir. In this implementation, the liquid level may be measured, because the capacitance of the liquid changes as the liquid level changes. Capacitive sensing provides a non-contact means for detecting the liquid level.

Level sensor 228 may output one or more signals indicating the measured liquid level to the controller in base unit 202. The occluder valve or a pinch valve disposed along tube 214 can be used to isolate the volume of liquid in auxiliary unit 204. In implementations where liquid only leaves system 200 when vaporized into the breathing gas in VTC 216, the controller can determine the amount of liquid vaporized (*i.e.,* the vaporization rate) or otherwise consumed (*i.e.,* consumption rate) over time. In some implementations, this information is used to determine the flowrate of liquid needed from external reservoir 210 in order to keep the liquid level at a steady state, where the liquid flowrate from external reservoir 210 equals the vaporization rate, in order to, for example, prevent auxiliary unit 204 and VTC 216 from drying up or ensure the breathing gas is kept at a constant vapor concentration (e.g., humidity). In other implementations, the liquid level is not at a steady state, but the controller determines, based on the vaporization flowrate, an appropriate time interval at which to allow a fixed volume of the liquid to enter the internal reservoir from external reservoir 210 that is approximately equal to the amount of liquid vaporized during that time interval.

From the calculated vaporization rate, the controller may also compute the humidity of the breathing gas exiting VTC 216 in implementations where the liquid is water. Another device may be used to determine the flowrate (mass flowrate and/or volumetric flowrate) of breathing gas in system 200. Such a device may be disposed in base unit 202 to measure breathing gas entering or exiting the blower, or the device may be disposed in auxiliary unit 204 to measure breathing gas flowrate entering or exiting auxiliary unit 204 or VTC 216. The device may alternatively be disposed along or within delivery tube 206 or patient connector 208 for a patient-proximate measurement of the breathing gas flowrate. In any of these cases, the device outputs one or more signals indicating the breathing gas flowrate to the controller. Also knowing the consumption rate or vaporization rate, the controller can calculate the humidity of breathing gas based on the liquid consumption/vaporization rate and the breathing gas flowrate, because liquid in the closed auxiliary unit 204 may only exit system 200 as vapor in the breathing gas when liquid flow from external reservoir 210 is stopped.

Knowing the humidity can serve several purposes. For example, an inappropriately low or high humidity may indicate that auxiliary unit 204, VTC 216, or another component of system 200 has become defective and requires replacement, allowing the controller to halt operation of system 200 and notify the operator before the patient is harmed. As another example, the performance of system 200 may be monitored over time, using humidity as a monitored variable, in order to detect deterioration and extend the usable lifetime of system 200. In this case, there is no need to limit all devices to the performance of outliers during lifetime testing. In addition, knowing humidity, adjustment of a temperature within VTC 216 relative to a temperature within delivery tube 206 can allow for adjustment of the humidity if the humidity output is monitored for safety. In some implementations, system 200 stores (*e.g.,* on a memory) a range of safe humidity levels for patient breathing gas, and the controller is configured to determine if the calculated humidity is within the safe range. If the calculated humidity falls outside of the safe range, the controller may adjust the power of pump 224 to adjust liquid flowrate and/or adjust the power of the blower to adjust breathing gas flowrate.

Latch 220 is configured to lock auxiliary unit 204 in recess 218 of base unit 202 when auxiliary unit 204 is correctly positioned/seated in recess 218 for proper operation of system 200 and interoperability between auxiliary unit 204 and base unit 202. A user may press against latch 220 to unlock auxiliary unit 204 and remove it from base unit 202. In order to ensure that auxiliary unit 204 is fully seated in recess 218 for proper operation, there may be one or more elements disposed in base unit 202 and auxiliary unit 204 configured to detect the presence, or the precise alignment of auxiliary unit 204 in recess 218. In some implementations, auxiliary unit 204 includes a RFID tag *(e.g.,* RFID tag 350 of FIG. 3) or other tag, and base unit 202 includes an antenna or reader *(e.g.,* RFID reader 351 of FIG. 3). The tag can be detected by the antenna or reader in a non-contact manner when the tag is positioned near the antenna or reader, essentially, when auxiliary unit 204 is seated in recess 218.

Accordingly, the tag may also include information that can be read by the antenna or reader and transmitted to the controller or a memory of base unit 202. For example, the tag may include information describing the type of or feature in auxiliary unit 204, such as low flow, high flow, aerosolization, humidification, oxygenation, nitric oxide, helium, and/or closed loop oxygen control. The tag may also include information relating to the state of the specific auxiliary unit 204 and/or its various components. For example, the information may include use history (including the in-use date), a shelf-life or remaining lifetime, embedded licensing, and/or recommended operating parameters (limit flowrates or humidity). The controller may use any of the information described above to determine and execute appropriate adjustments to system 200 and any of its various components. For example, depending on the type or features of auxiliary unit 204, certain operations or features may be enabled or disabled by the controller in accordance with the in-use auxiliary unit 204.

RFID tags or other tags can provide additional information about an auxiliary unit such as the auxiliary unit type, when it was first used, the expiration date, etc. This can overcome the drawbacks of the above-mentioned systems. In some systems, the device needs to be aware of a change in auxiliary unit even if the capital unit is in standby. To detect an auxiliary unit change in these systems RFID may require the device to use too much power while in standby. As an example, an active valve can close the air path when the auxiliary unit is removed and close the water fill path when an auxiliary unit is installed. Such features may not function correctly if the auxiliary unit is added or removed when the device is off or in a standby mode. This can occur if the auxiliary unit is set up in advance to be ready for a patient. The device is off when the auxiliary unit is installed and water may flow uncontrolled into the device. It would be beneficial for the device to power on and close the valve.

In some implementations, auxiliary unit 204 incudes one or more magnets (e.g., alignment marker 348 of FIG. 3), and base unit 202 includes a Hall effect sensor (e.g., alignment sensor 349 of FIG. 3) configured to sense the presence of the magnet in order to determine in a non-contact manner if auxiliary unit 204 is fully seated in recess 218. A Hall effect sensor measures a magnitude of a magnetic field and outputs a voltage proportional to the magnetic field magnitude. Accordingly, the Hall effect sensor may detect the magnetic field of the magnet when in close proximity. By using a magnet and Hall effect sensor, the precise position of auxiliary unit 204 may be detected, and a change in state may be detected even when system 200 is in a stand-by or low-power mode, because the Hall effect sensor may operate on a very low power when system 200 is in stand-by, and a change in this Hall effect sensor can be used to wake system 200 to, for example, read the RFID tag or start operation of certain components such as the blower or pump 224. As another example, the controller may be configured to close the occluder valve to block breathing gas flow from base unit 202 to auxiliary unit 204 when auxiliary unit 204 is removed, even if system 200 is in stand-by, to ensure that the gas path is not left open when the disposable is removed. The magnet may be configured to have a narrow range in which it can trigger the Hall effect sensor. The range may be less than about 5 mm, less than about 4 mm, less than about 3 mm, less than about 2 mm, or less than about 1 mm. In some implementations, the magnet is disposed within latch 220, so that the Hall effect sensor is triggered by the magnet when latch 220 is in the locked position. A barcode may be used in auxiliary unit 204 in addition to or in place of an RFID tag or other tag, such that a reader in base unit 202 reads the barcode when auxiliary unit 204 is docked in recess 218. The barcode may include the information about one or more components, as is described in relation to the RFID tag in this disclosure. As another option, base unit 202 includes an infrared (IR) proximity sensor having an IR beam positioned such that latch 220 breaks the beam, triggering the sensor, when auxiliary unit 204 is fully seated in recess 218. Other options for auxiliary unit presence sensing and data storage/transfer include quick response (QR) codes, global positioning system (GPS) chips, Bluetooth^{®}, Bluetooth^{®} low-energy, near-field communication (NFC), and pattern recognition matching.

System 200 may be configured to automatically switch to a stand-by or low-power mode under certain conditions. For example, system 200 may switch to stand-by mode when auxiliary unit 204 is not detected, when the blower is off and a user has not interacted with system 200 for a certain period of time, or when a user inputs a command to switch system 200 to the stand-by mode. When switching to stand-by or low-power mode, the controller may generate an alert to notify the user of the switch. System 200 may be configured to be powered by a standard wall outlet or a main battery. In either case, system 200 may include a reserve battery. The reserve battery may be a rechargeable battery, such as lithium-ion, lead-acid, nickel-cadmium, nickel-metal hydride, lithium-ion polymer, alkaline, or another equivalent. The reserve battery may be configured to provide power to system 200 when the wall outlet becomes unusable (e.g., during a power outage) or when the main battery requires replacement. In some implementations, system 200 can operate on solely the reserve battery for at least about 30 minutes, at least about 1 hour, at least about 2 hours, or at least about 5 hours. The stand-by or low-power mode may limit power usage of system 200 to a percentage of the maximum power usage of system 200 during normal operation (e.g., about 80%, about 70%, about 60%, about 50%, about 40%, about 30%, about 20%). In some implementations, the controller switches system 200 to the stand-by or low-power mode when the reserve battery is in use. When in stand-by or low-power mode, the controller may disable certain features, for example, to reduce power consumption. For example, the controller may disable heating elements or supplementary gas input, allowing operation of the blower and one or more alarms (e.g., auditory alarms, notifications on display 230).

System 200 may also include one or more temperature sensors, such as temperature sensors 342 and 344 of FIG. 3. In some implementations, base unit 202 includes one or more infrared (IR) temperature sensors configured to measure liquid temperatures in auxiliary unit 204 by non-contact means. One or more signals indicating the liquid temperature may be transmitted from the sensor(s) to the controller. The controller may be configured to determine if the measured temperature is within a stored safe temperature range. If the temperature is within the safe range, then the controller may alert the user by one or more alarms (e.g., auditory alarms, notifications on display 230) and/or change one or more controllable operational parameters to effect a change in the temperature. This may involve changing the blower flowrate, In some implementations, base unit 202 includes one or more IR temperature sensors configured to measure a temperature of the heater plate that is immersed in liquid in auxiliary unit 204, for example, to detect overheating of the heater plate. In some implementations, the heater plate includes a tab that protrudes from the top of the heating plate. The tab may be disposed such that it is the first part of the heating plate to be exposed (not immersed in liquid) when the liquid is at a low level. When the tab is not immersed, it will heat up quickly if the heating plate and heating element are in operation. A temperature sensor may be disposed to align with the tab when the auxiliary unit is seated, so that the temperature of the tab may be monitored by sending one or more signals indicating the tab temperature to the controller. When the tab heats up quickly during a low liquid level state of auxiliary unit 204, the temperature sensor can detect the rapid rise in temperature and alert the controller. In response to detecting a rapid rise in the tab temperature indicative of a low liquid level, the controller may be configured to turn off the heating element *(e.g.,* the induction coil). In response to detecting a rapid rise in the tab temperature indicative of a low liquid level, the controller may be configured to increase or open the liquid flow from external reservoir 210 by, for example, actuating the occluder valve or pump 224. In response to detecting a rapid rise in the tab temperature indicative of a low liquid level, the controller may be configured to completely shut off system 200 and/or generate an alarm. An alarm may be auditory or visual via display 230.

Delivery tube 206 conducts the breathing gas to the patient. Jacketed delivery tubes are typically smaller in inside diameter than heated wire delivery tubes and have fewer related issues. For example, the weight of liquid needed to properly heat a larger tube can provide a poor user experience if the tube diameter is increased too much, because the user may have difficulty moving a tube substantially weighed down by the liquid. Smaller jacketed delivery tubes can work when the pressure of the pressure source is high enough. In system 200, breathing gas is pressurized and supplied from the blower in base unit 202, without external pressurization in some implementations, so limited pressure may be available. With a blower, the tube diameter needs to be increased to account for a lower pressure source. It may be advantageous to size a cross-sectional diameter of delivery tube 206 to be as large as possible, in order to accommodate the lower back pressure while maintaining functionality. Generally, an increased diameter increases the weight of the delivery tube due to the larger volume of liquid required to heat the gas. Increasing the diameter can also lead to water accumulation in the delivery tube. Unlike the larger heated wire tubes, which must be tilted to drain liquid to the patient, the smaller jacketed tube may deliver water to the patient by simply increasing the flow above a certain threshold where the water may be conducted along the delivery tube by the flow of breathing gas. Accordingly, an upper limit of the diameter may be set based on, when operating at a minimum breathing gas flowrate, the velocity of breathing gas is sufficient to convey any liquid that has built-up in delivery tube 206 as rain-out to patient connector 208 where it may be collected and removed. In some implementations, the breathing gas flowrate is 5 L/min, and the delivery tube inner diameter is less than or equal to about 0.26 in. Delivery tube 206 may be constructed of a flexible material as to allow a user or operator to manipulate delivery tube 206 without obstructing breathing gas flow.

The increased diameter can also make the delivery tube more prone to kinking. Accordingly, delivery tube 206 may be constructed to be kink resistant. In some implementations, delivery tube 206 includes a jacket. As discussed above, heated liquid may be used to heat, in turn, breathing gas. For example, delivery tube 206 may include a jacket configured to receive heated liquid and convey the heated liquid around an inner lumen that conveys the breathing gas. Heated liquid may travel along a full length of delivery tube 206 in a first direction towards patient connector 208, and then return in an opposite direction back to auxiliary unit 204. In some implementations, the jacket includes a plurality of ribs extending in a radial direction through the jacket from a first conduit defining a breathing gas lumen to a second conduit, wherein the jacket is defined as the annular space between the first conduit and the second conduit. The radial ribs define two or more channels of the jacket through which liquid may flow. The radial ribs allow bending of delivery tube 206 in any direction without kinking by preventing collapse of the inner breathing gas conduit when delivery tube 206 is bent. In some implementations, under flexure of delivery tube 206, one or more of the channels may collapse, but at least some of the channels and the breathing gas lumen remain open to allow for liquid and breathing gas flow, respectively. The jacket having radial ribs may mimic a thick-walled tube to prevent collapse of the breathing gas lumen. In some implementations, the breathing gas lumen wall has a thickness proportional to the breathing gas lumen inside diameter. In some implementations, the outer wall, defining the jacket, has a thickness proportional to the outer diameter of delivery tube 206 minus the inner diameter of the breathing gas lumen. In some implementations, each radial rib has a width proportional to the wall thickness of the breathing gas lumen.

Display 230 may be configured to show one or more parameters of system 200. For example, display 230 shows any of: breathing gas flowrate, liquid vaporization/consumption rate, humidity, liquid level, breathing gas velocity, heating plate temperature, and liquid temperature. In some implementations, display 230 acts as an interface for both alerting the user of system parameters and for accepting user inputs. User inputs may include target values for any of these parameters. The user inputs may be transmitted to the controller, which is configured to adjust the parameters, for example, by actuating one or more of the components of system 200.

As described above in relation to FIG. 2, the liquid in systems described herein follows a flow path during operation of said systems. As there are several components that may utilize or act on the liquid, there may be an ordered flow path through which liquid flows and undergoes various state changes or manipulations.

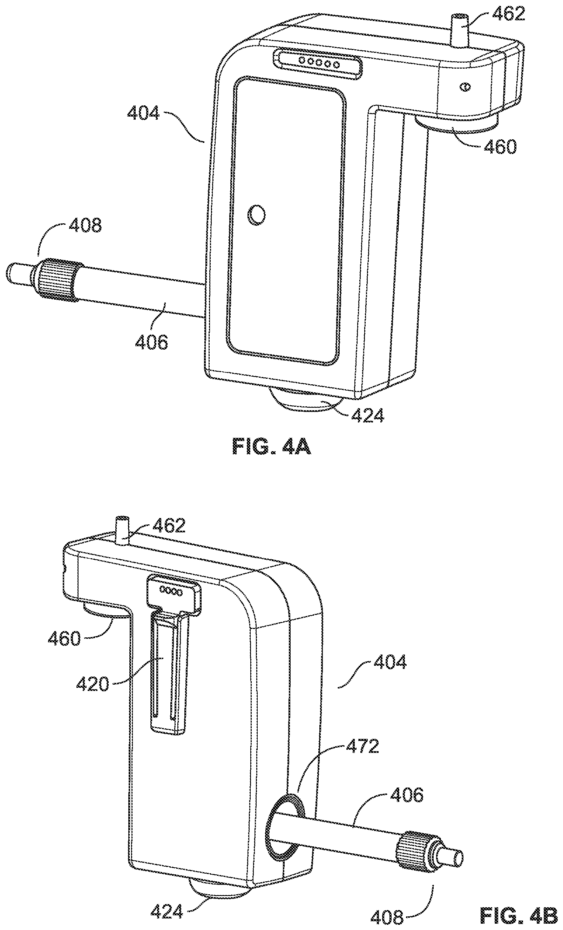

FIG. 3 is a cross-section of a respiratory therapy system 300 configured to output breathing gas to a patient, according to an illustrative implementation. System 300 includes a base unit 302 having a recess 318 in which auxiliary unit 304 is seated. Latch 320 locks auxiliary unit 304 in its current position. Liquid is stored in internal reservoir 332 of auxiliary unit 304. External reservoir 310 supplies liquid through external tube 314 to internal tube 315 of auxiliary unit 304. A valve 340 is disposed along external tube 314. Liquid flowing through internal tube 315 is conveyed into internal reservoir 332 through internal reservoir inlet 334. A vent 338 is disposed on internal reservoir 332. Liquid flows out of internal reservoir 332 and then alongside heating plate 327 immersed in the liquid. After heating, liquid flows into pump 324 and then out of pump outlet 346. Auxiliary unit 304 also includes an RFID tag 350 and an alignment marker 348. Base unit 302 includes a stator 322, a level sensor 328, a heat actuator 326, an RFID reader 351, an alignment sensor 349, and temperature sensors 342 and 344. System 300 and its components may combined with or modified by the systems and devices described in FIGs. 2, and 4A-4F.

The various components in base unit 302 may be configured to couple to corresponding components of auxiliary unit 304 in a non-contact manner. These components may be operatively coupled to a controller in base unit 302 for actuation and/or electronic communication. In some implementations, stator 322 is configured to magnetically couple to a rotor of pump 324. By magnetically coupling to the rotor, stator 322 may induce rotational motion in the rotor for non-contact control of pump 324. Heat actuator 326 may convey by non-contact means heat to or generate heat in heating plate 327 immersed in liquid. Heat actuator 326 may be operatively coupled to the controller to allow for indirect actuation of the rate at which heat is transferred to liquid surrounding heating plate 327. In some implementations, heat actuator 326 is a coil configured to generate a current in heating plate 327 via induction, the current generating heat due to a resistance in heating plate 327. The generated heat may transfer from immersed heating plate 327 to liquid on either side. In some implementations, level sensor 328 is a capacitive sensor configured to measure capacitance in internal reservoir 332. Internal reservoir 332 may have a constant cross-section 336, so the measured capacitance is indicative of a liquid level in internal reservoir 332. By utilizing capacitive sensing, the system may provide an early warning of low liquid levels and active control of the water level in the system. Measurements of flow rate and humidification may also be more accurate with the use of capacitive sensing to accurately measure remaining liquid water.

In some implementations, RFID reader 351 detects the presence of RFID tag 350 when auxiliary unit 304 is within recess 318. RFID reader 351 may read information stored on RFID tag 350. In some implementations, alignment marker 348 is disposed in latch 320, and alignment sensor 349 is configured to detect the presence of alignment marker 348 when latch 320 is in a locked position. Latch 320 in an unlocked position 321 is depicted in FIG. 3 by a dashed outline. In some implementations, alignment marker 348 is a magnet, and alignment sensor 349 is a Hall effect sensor configured to detect the magnetic field of the magnet. Alignment sensor 349 may transmit to the controller a signal indicating auxiliary unit 304 is fully seated in recess 318 when alignment marker 348 is detected. A barcode may be used in auxiliary unit 304 in addition to or in place of RFID tag 350, such that reader 351 in the base unit reads the barcode when auxiliary unit 304 is fully docked in recess 318 of base unit 302. The barcode may include the information about one or more components, as is described in relation to the RFID tag in this disclosure. As an alternative to alignment marker 348, the base unit may include an infrared (IR) proximity sensor having an IR beam positioned such that latch 320 breaks the beam, triggering the sensor, when auxiliary unit 304 is fully seated. In some implementations, temperature sensors 342 and 344 are IR temperature sensors configured to measure temperatures in auxiliary unit 304. For example, temperature sensor 342 may be positioned to measure a temperature of heating plate 327, and temperature sensor 344 may be positioned to measure a temperature of the liquid flowing away from heating plate 327.

Liquid may flow out of internal reservoir 332 to heating plate 327 and pump 324 due to hydrostatic pressure resulting from the height of internal reservoir 332 positioned above heating plate 327 and pump 324. In some implementations, pump 324 generates a suction pressure that draws liquid from internal reservoir 332, past heating plate 327, and into pump 324. Rotational motion of a rotor (not shown) in pump 324 may be used to convey the liquid along a flow path. The flow path may be partially enclosed in auxiliary unit 304. In some implementations, system 300 further includes a jacketed delivery tube (such as delivery tubes 206 and 406 of FIGs. 2, 4A, 4B, and 4D-4F), and the flow path may extend through the jacket of the delivery tube. In some implementations, pump outlet 346 is in fluid communication with the jacket, such that heated liquid is pumped into the jacket for heating of the breathing gas in an inner lumen of the delivery tube, wherein the inner lumen is concentrically surrounded by the jacket. Pump 324 may generate sufficient pressure such that liquid is conveyed through a first lumen of the jacket away in a direction away from auxiliary unit 304 and through a second lumen of the jacket in an opposite direction back to auxiliary unit 304. In some implementations, auxiliary unit 304 houses a vapor transfer cartridge (VTC), such as VTC's 216 and 416 of FIGs. 2 and 4, and liquid is conveyed from the delivery tube jacket into VTC for humidification of the breathing gas.

In some implementations, pump 324 conveys the liquid in a cyclic manner, in which liquid outputted from pump outlet 346 is pumped first through the delivery tube jacket, then through the VTC, into internal reservoir 332 *(e.g.,* through a recycled liquid reservoir inlet not shown), and finally past heating plate 327 before returning into pump 324. In this case, pump 324 generates enough pressure to convey the liquid through an entire cycle of this flow path for the liquid to return to pump 324. This ordered flow path may define a cycle or loop of fluid flow. This loop may be a closed loop, for example, if valve 340 is closed to prevent fluid flow through external tube 314. The loop may be sealed such that, when the loop is closed, liquid only leaves the loop as vapor in the VTC. In some implementations, the volume of liquid in the loop is at a steady state such that the rate of liquid leaving the loop as vapor in the VTC is equal to the rate of liquid entering the loop through tubes 314 and 315 from external reservoir 310. In other implementations, discrete volumes of the liquid are conveyed from external reservoir 310 through tubes 314 and 315 to the internal reservoir 332 at time intervals, such that the discrete volume for a given time interval is equal to the amount of liquid vaporized during that time interval.

Valve 340 may be used to isolate the volume of liquid in the loop, for example, by closing valve 340 to stop all liquid flow through tubes 314 and 315 from external reservoir 310. In some implementations, level sensor 328 outputs one or more signals to the controller in base unit 302 indicative of the liquid level in internal reservoir 332. Internal reservoir 332 may have constant cross-section 336, such that liquid level may be directly proportional to the volume of liquid in internal reservoir 332. When liquid flow is occluded by valve 340, liquid may only exit the loop by vaporization in the VTC. The controller can determine the vaporization rate (or consumption rate of liquid by the VTC) based on a change in liquid level in internal reservoir 332 over a period. If the controller also knows the breathing gas flowrate, for example, by receiving one or more signals indicating the flowrate, then the controller can be configured to determine the vapor content or humidity of the breathing gas using the breathing gas flowrate measurement(s) and the liquid level measurements. The absolute humidity can also be determined by the controller by also taking into account temperature measurements, for example, indicated by one or more signals transmitted to the controller by temperature sensors configured to measure the temperature of the breathing gas in the VTC, at the VTC exit, or in the delivery tube

While external valves such as valve 340 can be used to control liquid flow from an external reservoir, it can be advantageous have a valve built into an auxiliary unit to, for example, allow for optimized control of the liquid flow by a controller. Furthermore, as the use of breathing gas and liquid is intertwined, a built-in valve that provides simultaneous control over breathing gas and liquid flows may enable further optimization and ease of control.

In accordance with the implementations described above and in the following, an auxiliary unit for a respiratory therapy system is provided herein. FIGs. 4A-4F show multiple view angle and cross-sections of an auxiliary unit 404, according to an illustrative implementation. Auxiliary unit 404 may be implemented, for example, in the respiratory therapy systems 200 and 300 of FIGs. 2 and 3.

FIG. 4A shows auxiliary unit 404 having a pump 424, a gas seal 460, and an external tube connector 462, according to an illustrative implementation. A delivery tube 406 is connected to auxiliary unit 404, and a patient connector 408 is disposed at a distal end of delivery tube 406. Auxiliary unit 404 is configured to be seated in a base unit (not shown) of a respiratory therapy system, which is configured to provide pressurized breathing gas from a blower in the base unit to auxiliary unit 404 during operation. Base unit 202 of FIG. 2 is an example of a base unit that may receive auxiliary unit 404. Gas seal 660, which is part of an occluder valve, forms a sealed flow path for the breathing gas to enter auxiliary unit 404 from the base unit when auxiliary unit 404 is seated in the base unit. External tube connector 462 is configured to connect an external tube (not shown) that provides liquid to auxiliary unit 404 from an external reservoir (not shown). For example, external tube 214 and external reservoir 210 of FIG. 2 may be used in this implementation. Pump 424 is configured to convey the liquid from within auxiliary unit 404.

Delivery tube 406 is configured to receive a flow of breathing gas from auxiliary unit 404 and convey the breathing gas to a patient, through patient connector 408 at the distal end of delivery tube 406. In some implementations, a nasal cannula is connected to patient connector 408 to provide the breathing gas into at least one nare of the patient. The breathing gas may be humidified with the liquid by a VTC in auxiliary unit 404. Pump 424 may convey the liquid along a flow path, including through the VTC in which at least a portion the liquid may be vaporized into the breathing gas, which is simultaneously passed through the VTC. When auxiliary unit 404 is seated in the base unit, pump 424 may couple to a pump actuator in the base unit, and the pump actuator may be operatively coupled to a controller, which may control the power supplied to pump 424. For example, pump 424 may include a rotor, which is configured to magnetically couple to a stator in the base unit. Through the magnetic coupling, rotational motion of the rotor is generated and used to convey fluid along the flow path. In some implementations, the flow path includes a jacket of delivery tube 406.

FIG. 4B shows an alternate view angle of auxiliary unit 404. From this angle, a latch 420 is shown on the side of auxiliary unit 404, according to an illustrative implementation. Latch 420 locks auxiliary unit 404 into the base unit when auxiliary unit 404 is fully seated. Delivery tube 406 is connected to auxiliary unit outlet 472.

Latch 420 allows auxiliary unit 404 to be locked and unlocked from its fully seated position in the base unit. Latch 420 may enable locking of auxiliary unit 404 by having a ridge that resides in a notch in the base unit. A user or operator may press against latch 420 to release latch 420 from the notch and unlock auxiliary unit 404 when removing it from the base unit. Latch 420 may have one or more components embedded in it. For example, a marker, such as alignment marker 348 of FIG. 3, may be embedded in latch 420, and the base unit may include a sensor, such as alignment sensor 349 of FIG. 3, configured to detect the presence of the marker when auxiliary unit 404 is fully seated in the base unit and latch 420 is locked. In some implementations, the marker is a magnet, and the sensor is a Hall effect sensor configured to detect the magnetic field of the magnet. In some implementations, latch 420 includes an RFID tag, such as RFID tag 350 of FIG. 3, and the base unit includes an RFID reader, such as RFID reader 351 of FIG. 3, configured to detect the presence of the RFID tag and read information stored on the RFID tag. The information on the RFID tag may identify auxiliary unit 404 as having certain functionalities, may provide use history of auxiliary unit 404, or may provide recommended system parameters such as flowrates, humidity level, and temperature. A barcode may be used in auxiliary unit 404 in addition to or in place of an RFID tag or other tag, or magnet, such that a reader in the base unit reads the barcode when auxiliary unit 404 is fully docked in the recess of the base unit. The barcode may include the information about one or more components, as is described in relation to the tags in this disclosure. As another option, the base unit includes an infrared (IR) proximity sensor having an IR beam positioned such that latch 420 breaks the beam, triggering the sensor, when auxiliary unit 404 is fully seated. Other options for auxiliary unit presence sensing include quick response (QR) codes, global positioning system (GPS) chips, Bluetooth^{®}, Bluetooth^{®} low-energy, near-field communication (NFC), and pattern recognition matching.

Auxiliary unit outlet 472 functions as a port for connection of delivery tube 406 to auxiliary unit 404 for transmission of the breathing gas. Outlet 472 may have a shape such that delivery tube 406 can bend and rotate at outlet 472 without being kinked by the edges of outlet 472. The shape of outlet 472 may also prevent dislodging of delivery tube 406 from auxiliary unit 404. In some implementations, outlet 472 is cone shaped. In some implementations, outlet 472 is chamfered. In some implementations, outlet 472 is filleted. Outlet 472 may be configured to have no hard edges that may kink or damage delivery tube 406; for example, outlet 472 may be rounded.

FIG. 4C shows a cross-section of auxiliary unit 404 which includes, in addition to the components previously described, a VTC 416, a heating plate 427, an internal reservoir 432, and a delivery connector 468, according to an illustrative implementation. Liquid is provided to internal reservoir 432 at reservoir inlet 434, which receives liquid from an internal tube (not shown), for example, internal tube 315 of FIG. 3. Liquid flows out from internal reservoir 432 past heating plate 427, which is immersed in the liquid during operation. Liquid is drawn into pump 424, which expels liquid through pump outlet 446. VTC 416 has a first cap 464 configured to receive breathing gas and a second cap 465 configured to output breathing gas to delivery connector 468. VTC 416 also includes VTC liquid inlet 466 and VTC liquid outlet 467. Delivery connector 468 is configured to convey breathing gas from second cap 465 of VTC 416 to delivery tube 406 through connector gas outlet 469. Delivery connector 468 further includes connector liquid outlet 470 and connector liquid inlet 471.

Liquid entering auxiliary unit 404 through liquid tube connector 462 is directed to reservoir inlet 434 by an internal tube (not shown), such as internal tube 315 of FIG. 3. Liquid stored in internal reservoir 432 may flow out of internal reservoir 432 to a heating section where heating plate 427 is immersed in liquid. In some implementations, liquid flows into the heating section due to hydrostatic pressure and/or gravitational flow, a result of internal reservoir 432 being positioned above the heating section. In some implementations, pump 424 generates a suction pressure that draws liquid out of internal reservoir 432 and into the heating section before being drawn into pump 424. When auxiliary unit 404 is seated in the base unit, heating plate 427 may be coupled to a heat actuator in the base unit. In some implementations, the heat actuator is a coil configured to generate a current in the heating plate via induction, and the current produces heat due to a resistance in the heating plate.

Liquid flows or is drawn into pump 424 from the heating section. The pump expels liquid out of pump outlet 446, which is in fluid communication with connector liquid outlet 470 of delivery connector 468. Delivery connector 468 may be an overmolded rubber piece having one or more lumens. Connector liquid inlet 470 conveys pumped, heated liquid from pump outlet 446 into a jacket of delivery tube 406. The jacket concentrically surrounds an inner gas conduit through which breathing gas is conveyed, such that the heated liquid flowing through the jacket insulates the breathing gas in the inner gas conduit. This feature may be advantageous as to maintain a sufficiently high breathing gas temperature in order to prevent rain-out of vapor in the breathing gas. Rain-out occurs when vapor in the breathing gas condenses into its liquid form during gas delivery. This condensation can cause obstruction of delivery tube 406, patient connector 408, or a nasal cannula connected to patient connector 408. The heated liquid travels in two directions in the jacket, and the jacket accordingly includes at least two lumens or channels, where each lumen or channel connects to either connector liquid outlet 470 or connector liquid inlet 471. For example, liquid is pumped through connector liquid outlet 470 into a first jacket channel, which conveys the liquid along delivery tube 406 in a first direction towards patient connector 408. At the distal end of delivery tube 406, where patient connector 408 is disposed, the liquid may change directions and enter a second channel which conveys the liquid along delivery tube 406 in a second direction which is substantially opposite of the first direction. The second channel conveys the liquid into connector liquid inlet 471 of delivery connector 468.

Connector liquid inlet 471 is in fluid communication with VTC liquid inlet 466 in order to convey the fluid from the jacket to VTC 416. Within VTC 416, liquid may be vaporized into the breathing gas conveyed into VTC 416 through first cap 464. For example, the liquid may be water, and VTC 416 may output from second cap 465 a flow of humidified breathing gas containing water vapor. For vaporization to occur, the temperature in VTC 416 may be at or near the gas therapy temperature. Heating plate 427 may heat the liquid to a sufficiently high temperature, e.g., about 100 °F. VTC 416 may contain a plurality of fibers, which allow vapor to pass from the liquid flow into the breathing gas flow. Liquid that is not vaporized in VTC 416 may flow out of VTC liquid outlet 467, which returns the liquid to internal reservoir 432.

Breathing gas exiting second cap 465 of VTC 416 is directed through connector gas outlet 469 of delivery connector 468 into delivery tube 406. The breathing gas, which may be humidified or contain vapor, flows through the inner gas conduit of delivery tube 406 and is insulated or heated by liquid flowing through the jacket. At patient connector 408, the breathing gas may be directed directly to the patient or into one or more additional devices, such as a face mask or a nasal cannula.

FIG. 4D is cross-sectional view of auxiliary unit 404, according to an illustrative implementation. In this view, the occluder valve 452 is shown in auxiliary unit 404. Delivery tube 406 is connected to delivery connector 468 at auxiliary unit outlet 472. Occluder valve 452 receives liquid flow through external tube connector 462. A flexible portion 456 includes a liquid path valve seal 457 disposed below valve liquid inlet 658. Internal tube 415 is in fluid communication with valve 452 and internal reservoir 432. Gas path 412 is in fluid communication with an annular space formed by gas seal 660 and with first cap 464 of VTC 416.

Occluder valve 452 is configured to mate with the base unit when auxiliary unit 404 is fully seated. The base unit may include a controller, an actuator, and a gas path valve seal. The controller operates the actuator to adjust a position of the gas path valve seal in order to control breathing gas flow from the base unit into auxiliary unit 404. The gas path valve seal may be raised against the liquid path valve seal 457 in order to simultaneously control liquid flowrate through occluder valve 452, because liquid path valve seal 457 is a rigid piece joined to flexible portion which may be flexed to raise liquid path valve seal 457 to narrow a gap between liquid path valve seal 457 and valve liquid inlet 458. The actuator, gas path valve seal, and liquid path valve seal may have, but are not limited to, three positions, which are summarized in Table 3. The controller may operate the actuator to switch between each of these positions based on system inputs or received signals. Breathing gas may be directed by valve 452 into gas path 412, which conveys breathing gas into first cap 464 of VTC 416. Liquid may be directed by valve 452 into internal tube 415, which conveys liquid into internal reservoir 432.

Delivery tube 406 is connected to delivery connector 468 at auxiliary unit outlet 472 for receiving breathing gas and liquid flows. Delivery tube 406 includes an inner gas conduit 407 configured to receive breathing gas from second cap 465 of VTC 416 through connector gas outlet 469 of delivery connector 468. Delivery tube 406 includes a first jacket channel 474 configured to receive liquid from pump outlet 464 through connector liquid outlet 470 of delivery connector 468. Liquid flows through first jacket channel 474 and a second jacket channel 475, for example, to heat or insulate the breathing gas in inner gas conduit 407. In some implementations, first jacket channel 474 conveys liquid in a first direction away from auxiliary unit outlet 472 and towards patient connector 408. First and second jacket channels 474 and 475 each have distal ends disposed within patient connector 408, which may allow for fluid communication between first and second jacket channels 474 and 475. For example, the liquid flows in the first direction through first jacket channel 474, and, upon reaching patient connector 408, flows out of the distal end of first jacket channel 474 and into the distal end of second jacket channel 475. Liquid may then flow through second jacket channel 475 in a second direction which may be substantially opposite of the first direction. Second jacket channel 475 conveys the liquid back into delivery connector 468 through connector liquid inlet 471. In some implementations, first jacket channel 474 covers a first portion of inner gas conduit 407, the first portion having an approximately semi-circular cross-sectional shape. Second jacket channel 475 may cover a second portion on the opposite side of inner gas conduit 407, the second portion having an approximately semi-circular cross-sectional shape. Auxiliary outlet 472 in this implementation has a rounded (filleted) shape, which may prevent kinking of delivery tube 406, and occlusion of inner gas conduit 407, first jacket channel 474, and second jacket channel 475 when delivery tube 406 is bended or flexed.

FIGs. 4E and 4F show different cross-sections of a lower portion of auxiliary unit 404, according to an illustrative implementation. In FIG. 4E, delivery tube 406 is connected to auxiliary unit 404 at auxiliary outlet 472, and a cross-section of delivery tube 406 is shown. First jacket channel 474 includes four sub-channels 474a, 474b, 474c, and 474d. Second jacket channel 475 includes four sub-channels 475a, 475b, 475c, and 475d. The plurality of sub-channels surround inner gas conduit 407.

First jacket channel 474 and second jacket channel 475 are divided into sub-channels 474a-d and 475a-d by a plurality of radial ribs. Each radial rib extends from an inner wall defining inner gas conduit 407 to an outer wall of delivery tube 406, wherein the annular space between the inner wall and outer wall defines the jacket. Each pair of radial ribs defines one sub-channel of the jacket through which liquid flows. The radial ribs allow bending of delivery tube 406 in any direction without kinking by preventing collapse of inner gas conduit 407 when delivery tube 406 is bent. In some implementations, under flexure of delivery tube 406, one or more of the sub-channels 474a-d and 475a-d may collapse or become occluded, but at least some of the sub-channels 474a-d and 475a-d and inner gas conduit 407 remain open to allow for liquid and breathing gas flow, respectively. The jacket having radial ribs may mimic a thick-walled tube to prevent collapse of inner gas conduit 407. In some implementations, the inner wall defining inner gas conduit 407 has a thickness proportional to an internal diameter of inner gas conduit 407. In some implementations, the outer wall, defining the jacket, has a thickness proportional to the overall diameter of delivery tube 406 minus the internal diameter of inner gas conduit 407. In some implementations, each radial rib has a width proportional to the inner wall thickness.

In FIG. 4F, a cross-section of auxiliary unit 404 shows delivery tube 406 disposed in delivery connector 468. Sub-channels 474a, 474b, 474c, and 474d of first jacket channel 474 of delivery tube 406 are in fluid communication with connector liquid outlet 470 of delivery connector 468. Sub-channels 475a, 475b, 475c, and 475d of second jacket channel 475 of delivery tube 406 are in fluid communication with connector liquid inlet 471 of delivery connector 468. A tab 476 disposed at the top of heating plate 427.

As described above, first jacket channel 474 is in fluid communication with connector liquid outlet 470 in order to receive liquid from pump outlet 446. Liquid is flowed or pumped through the sub-channels 474a-d and then through sub-channels 475a-d of second jacket channel 475. Inner gas conduit 407 is in fluid communication with connector gas outlet 469 to receive breathing gas from VTC second cap 465.

Tab 476 is disposed at the top of heating plate 427, near internal reservoir 432. In some implementations, tab 476 is disposed at the top of heating plate 427 such that, when liquid in internal reservoir 432 reaches a low enough level, tab 476 is exposed while the rest of heating plate 427 remains immersed in liquid. When auxiliary unit 404 is fully seated in the base unit, a temperature sensor (such as temperature sensor 342 of FIG. 3) may be positioned such that it aligns with tab 476 and can measure the temperature of tab 476. When tab 476 is not immersed in liquid, it will heat up quickly if the heating plate and heating element are in operation. The temperature of the tab may be monitored by sending one or more signals indicating the tab temperature to the controller. When the tab heats up quickly during a low liquid level state of auxiliary unit 404, the temperature sensor can detect the rapid rise in temperature and alert the controller. In response to detecting a rapid rise in the tab temperature indicative of a low liquid level, the controller may be configured to turn off the heat actuator (e.g., an induction coil). In response to detecting a rapid rise in the tab temperature indicative of a low liquid level, the controller may be configured to increase or open the liquid flow from the external reservoir by, for example, actuating occluder valve 452 from the extended position to the middle position to allow liquid flow to refill internal reservoir 432. In response to detecting a rapid rise in the tab temperature indicative of a low liquid level, the controller may be configured to completely shut off the respiratory therapy system. In some implementations, the base unit includes a spring element that may be actuated by the controller to auto-eject auxiliary unit 404 under certain conditions, for example, when a rapid temperature rise is indicated.

It should be understood that the systems and devices shown in FIGs. 2, 3, and 4A-4F are examples of settings in which the humidification control may be performed, but the humidification control may be performed on other systems involving humidification of breathing gas.

Variations and modifications will occur to those of skill in the art after reviewing this disclosure.

The systems and methods described may be implemented locally on a respiratory therapy system or a controller of a respiratory therapy system. The respiratory therapy system may include a data processing apparatus. The systems and methods described herein may be implemented using a remote, , separate data processing apparatus connected directly or indirectly through cloud applications. Communication with such a separate data processing apparatus may be in real-time (or near real-time).

**In** general, embodiments of the subject matter and the functional operations described in this specification can be implemented in digital electronic circuitry, or in computer software, firmware, or hardware, including the structures disclosed in this specification and their structural equivalents, or in combinations of one or more of them. The methods may be implemented using one or more computer program products, i.e., one or more modules of computer program instructions encoded on a computer readable medium for execution by, or to control the operation of, data processing apparatus. The computer readable medium can be a machine-readable storage device, a machine-readable storage substrate, a memory device, a composition of matter affecting a machine-readable propagated signal, or a combination of one or more of them. The term "data processing apparatus" encompasses all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, or a combination of one or more of them. A propagated signal is an artificially generated signal, e.g., a machine-generated electrical, optical, or electromagnetic signal that is generated to encode information for transmission to suitable receiver apparatus.

A computer program (also known as a program, software, software application, script, or code) can be written in any form of programming language, including compiled or interpreted languages, and it can be deployed in any form, including as a stand-alone program or as a module, component, subroutine, or other unit suitable for use in a computing environment. A computer program may correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform functions by operating on input data and generating output. The processes and logic flows can also be performed by, and apparatus can also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices.

Examples of changes, substitutions, and alterations are ascertainable by one skilled in the art that could be made without departing from the scope of the present disclosure.

The scope of the present invention is defined by the following appended claims.

## Claims

1. A respiratory therapy system (200, 300) comprising:
a gas supply module (202, 302) configured to supply a flow of breathing gas;
a liquid supply module configured to supply a flow of liquid, the liquid supply module comprising:
a heater (327, 427) configured to heat the liquid; and
a pump (224, 324, 424) configured to circulate the liquid; and
a vapor transfer module configured to receive the flow of breathing gas and liquid, humidify the breathing gas, and output heated and humidified breathing gas, wherein the vapor transfer module comprises a vapor transfer cartridge (216, 416) and a delivery tube (206, 406), the vapor transfer cartridge (216, 416) comprising:
a gas inlet (464) configured to receive the flow of breathing gas from the gas supply module;
a gas outlet (465) configured to output the heated and humidified breathing gas;
a liquid inlet (466); and
a liquid outlet (467);
wherein the gas inlet (464) and the liquid outlet (467) are disposed at a first end of the vapor transfer cartridge (216, 416), and the gas outlet (465) and the liquid inlet (466) are disposed at a second end of the vapor transfer cartridge (216, 416), the second end of the vapor transfer cartridge (216, 416) being opposite to the first end of the vapor transfer cartridge (216, 416) such that the liquid and the breathing gas flow through the vapor transfer cartridge (216) in opposite directions;
the system (200, 300) being configured to:
supply the flow of breathing gas from the gas supply module to the vapor transfer module;
supply, for heating the flow of breathing gas, the flow of liquid from the liquid supply module to the vapor transfer module at a first liquid flowrate;
output, from the gas outlet (465), the heated and humidified breathing gas from the vapor transfer module at a first gas temperature, a first gas flowrate, and a first humidity;
adjust the first liquid flowrate to a second liquid flowrate, the liquid temperature
at the liquid inlet being held constant before and after the adjustment; and
output the heated and humidified gas from the vapor transfer module at about the first gas temperature, about the first gas flowrate, and a second humidity;
wherein a difference between the first humidity and second humidity is inversely proportional to the adjustment of the first liquid flowrate to the second liquid flowrate.

2. The system (200, 300) of claim 1, wherein the liquid supply module comprises a reservoir (332, 432) configured to hold liquid and has an outlet port and a return port, the return port being in fluid communication with the vapor transfer module and configured to convey liquid from the vapor transfer module to the reservoir (332, 432).

3. The system (200, 300) of claim 2, wherein:
the liquid outlet of the vapor transfer cartridge (216, 416) is configured to convey liquid from the vapor transfer cartridge (216, 416) to the reservoir (332, 432) via the return port.

4. The system (200, 300) of any of the preceding claims, wherein the delivery tube (206, 406) comprises:
a gas lumen (407); and
a liquid lumen (474, 475) at least partially surrounding the gas lumen (407), the gas lumen (407) being in fluid communication with the gas outlet of the vapor transfer cartridge (216, 416) so as to receive the heated and humidified breathing gas from the gas outlet of the vapor transfer cartridge (216, 416).

5. The system (200, 300) of claim 4, wherein the vapor transfer module and the liquid supply module define a liquid circulation loop, whereby liquid flows from the reservoir (332, 432), to the heater (327, 427), to the liquid lumen (474, 475) of the delivery tube (206, 406), to the liquid inlet of the vapor transfer cartridge (216), through the vapor transfer cartridge (216), and to the reservoir (332) via the return port.

6. The system (200, 300) of claim 4 or 5, wherein:
the delivery tube (406) comprises:
a first end; and
a second end, wherein the gas lumen (407) receives the heated and humidified breathing gas at the first end of the delivery tube (406) and conveys the heated and humidified gas to the second end of the delivery tube (406); and
the liquid lumen (474, 475) comprises:
a first channel (474) configured to convey liquid from the first end of the delivery tube (406) to the second end of the delivery tube (406); and
a second channel (475) in fluid communication with the first channel (474) at the second end of the delivery tube (406) and configured to convey the liquid from the second end of the delivery tube (406) to the first end of the delivery tube (406).

7. The system (200, 300) of claim 6, wherein the second channel (475) at the first end of the delivery tube (406) is in fluid communication with the liquid inlet of the vapor transfer cartridge (416), and the liquid inlet receives liquid from the second channel (475).

8. The system of claim 6, wherein the delivery tube further comprises a first temperature sensor disposed at an inlet of the first channel and a second temperature sensor disposed at an outlet of the second channel.

9. The system of claim 8, wherein the first temperature sensor and second temperature sensor each measure a temperature, and a temperature difference between the measured temperatures is less than 5% of the first gas temperature.

10. The system (200, 300) of any of the preceding claims, further comprising a nasal cannula coupled to the delivery tube (206, 406) and configured to convey the heated and humidified breathing gas to nares of a patient.

11. The system of any of the preceding claims, wherein the vapor transfer cartridge comprises:
a plurality of hollow fibers;
a pleated membrane; or
a flat sheet membrane.

12. The system of any of the preceding claims, wherein the vapor transfer cartridge comprises a heated wick.

13. The system of any of the preceding claims, wherein:
the gas supply module comprises a blower configured to output the flow of breathing gas; and/or
the system (200, 300) further comprises: a controller operatively coupled to the gas supply module and the liquid supply module and configured to adjust at least one of a gas temperature setpoint, a gas flowrate setpoint, a liquid flowrate setpoint, or a liquid temperature setpoint.

14. The system (200, 300) of any of the preceding claims, wherein:
the second liquid flowrate is less than the first liquid flowrate, and the second humidity is greater than the first humidity; or
the second liquid flowrate is greater than the first liquid flowrate, and the second humidity is less than the first humidity.

15. The system (200, 300) of any of the preceding claims, wherein at least one of:
the first gas temperature is 30 to 40 °C;
the first gas flowrate is 5 to 45 L/min;
at least one of the first humidity or the second humidity is 25-45 mg/L.

## Patentansprüche

1. Atemtherapiesystem (200, 300), umfassend:
ein Gaszufuhrmodul (202, 302), das konfiguriert ist, um einen Fluss von Atemgas zu liefern;
ein Flüssigkeitsversorgungsmodul, das konfiguriert ist, um einen Flüssigkeitsfluss zu liefern, das Flüssigkeitsversorgungsmodul umfassend:
eine Heizung (327, 427), die konfiguriert ist, um die Flüssigkeit zu erhitzen; und
eine Pumpe (224, 324, 424), die konfiguriert ist, um die Flüssigkeit umzuwälzen; und
ein Dampfübertragungsmodul, das konfiguriert ist, um den Fluss von Atemgas und Flüssigkeit zu empfangen, das Atemgas zu befeuchten und erhitztes und befeuchtetes Atemgas auszugeben, wobei das Dampfübertragungsmodul eine Dampfübertragungspatrone (216, 416) und ein Abgaberohr (206, 406) umfasst, die Dampfübertragungspatrone (216, 416) umfassend:
einen Gaseinlass (464), der konfiguriert ist, um den Fluss von Atemgas von dem Gaszufuhrmodul zu empfangen;
einen Gasauslass (465), der konfiguriert ist, um das erwärmte und befeuchtete Atemgas auszugeben;
einen Flüssigkeitseinlass (466); und
einen Flüssigkeitsauslass (467);
wobei der Gaseinlass (464) und der Flüssigkeitsauslass (467) an einem ersten Ende der Dampfübertragungspatrone (216, 416) angeordnet sind und der Gasauslass (465) und der Flüssigkeitseinlass (466) an einem zweiten Ende der Dampfübertragungspatrone (216, 416) angeordnet sind, wobei das zweite Ende der Dampfübertragungspatrone (216, 416) dem ersten Ende der Dampfübertragungspatrone (216, 416) gegenüberliegt, sodass die Flüssigkeit und das Atemgas in entgegengesetzten Richtungen durch die Dampfübertragungspatrone (216) fließen;
das System (200, 300) konfiguriert ist zum:
Zuführen des Flusses von Atemgas vom Gaszufuhrmodul zum Dampfübertragungsmodul;
Zuführen des Flusses der Flüssigkeit vom Flüssigkeitszufuhrmodul zum Dampfübertragungsmodul mit einer ersten Flüssigkeitsflussrate, um den Fluss von Atemgas zu erhitzen;
Ausgeben des erhitzten und befeuchteten Atemgases aus dem Dampfübertragungsmodul mit einer ersten Gastemperatur, einer ersten Gasflussrate und einer ersten Feuchtigkeit über den Gasauslass (465);
Einstellen der ersten Flüssigkeitsdurchflussrate auf eine zweite Flüssigkeitsdurchflussrate, wobei die Flüssigkeitstemperatur am Flüssigkeitseinlass vor und nach der Einstellung konstant gehalten wird; und Ausgeben des erhitzten und befeuchteten Gases aus dem Dampfübertragungsmodul bei etwa der ersten Gastemperatur, etwa der ersten Gasdurchflussrate und einer zweiten Feuchtigkeit;
wobei eine Differenz zwischen der ersten Luftfeuchtigkeit und der zweiten Luftfeuchtigkeit umgekehrt proportional zur Anpassung der ersten Flüssigkeitsdurchflussrate an die zweite Flüssigkeitsdurchflussrate ist.

2. System (200, 300) nach Anspruch 1, wobei das Flüssigkeitsversorgungsmodul ein Reservoir (332, 432) umfasst, das konfiguriert ist, um Flüssigkeit aufzunehmen, und einen Auslassanschluss und einen Rückführanschluss aufweist, wobei der Rückführanschluss in Fluidverbindung mit dem Dampfübertragungsmodul steht und konfiguriert ist, um Flüssigkeit von dem Dampfübertragungsmodul zu dem Reservoir (332, 432) zu befördern.

3. System (200, 300) nach Anspruch 2, wobei:
der Flüssigkeitsauslass der Dampfübertragungspatrone (216, 416) konfiguriert ist, um Flüssigkeit aus der Dampfübertragungspatrone (216, 416) über den Rücklaufanschluss zum Reservoir (332, 432) zu befördern.

4. System (200, 300) nach einem der vorstehenden Ansprüche, wobei das Abgaberohr (206, 406) Folgendes umfasst:
ein Gaslumen (407); und
ein Flüssigkeitslumen (474, 475), das das Gaslumen (407) mindestens teilweise umgibt, wobei das Gaslumen (407) in Fluidverbindung mit dem Gasauslass der Dampfübertragungspatrone (216, 416) steht, um das erhitzte und befeuchtete Atemgas vom Gasauslass der Dampfübertragungspatrone (216, 416) zu empfangen.

5. System (200, 300) nach Anspruch 4, wobei das Dampfübertragungsmodul und das Flüssigkeitszufuhrmodul eine Flüssigkeitszirkulationsschleife definieren, wodurch Flüssigkeit vom Reservoir (332, 432) zur Heizung (327, 427), zum Flüssigkeitslumen (474, 475) des Zufuhrrohrs (206, 406), zum Flüssigkeitseinlass der Dampfübertragungspatrone (216), durch die Dampfübertragungspatrone (216) und über den Rücklaufanschluss zum Reservoir (332) fließt.

6. System (200, 300) nach Anspruch 4 oder 5, wobei:
das Abgaberohr (406) Folgendes umfasst:
ein erstes Ende; und
ein zweites Ende, wobei das Gaslumen (407) das erhitzte und befeuchtete Atemgas am ersten Ende des Abgaberohrs (406) empfängt und das erhitzte und befeuchtete Gas zum zweiten Ende des Abgaberohrs (406) befördert; und
das Flüssigkeitslumen (474, 475) Folgendes umfasst:
einen ersten Kanal (474), der konfiguriert ist, um Flüssigkeit vom ersten Ende des Abgaberohrs (406) zum zweiten Ende des Abgaberohrs (406) zu befördern; und
einen zweiten Kanal (475), der am zweiten Ende des Abgaberohrs (406) in Fluidverbindung mit dem ersten Kanal (474) steht und konfiguriert ist, um die Flüssigkeit vom zweiten Ende des Abgaberohrs (406) zum ersten Ende des Abgaberohrs (406) zu fördern.

7. System (200, 300) nach Anspruch 6, wobei der zweite Kanal (475) am ersten Ende des Abgaberohrs (406) in Fluidverbindung mit dem Flüssigkeitseinlass der Dampfübertragungspatrone (416) steht und der Flüssigkeitseinlass Flüssigkeit aus dem zweiten Kanal (475) empfängt.

8. System nach Anspruch 6, wobei das Abgaberohr ferner einen ersten Temperatursensor umfasst, der an einem Einlass des ersten Kanals angeordnet ist, und einen zweiten Temperatursensor, der an einem Auslass des zweiten Kanals angeordnet ist.

9. System nach Anspruch 8, wobei der erste Temperatursensor und der zweite Temperatursensor jeweils eine Temperatur messen und ein Temperaturunterschied zwischen den gemessenen Temperaturen weniger als 5 % der ersten Gastemperatur beträgt.

10. System (200, 300) nach einem der vorstehenden Ansprüche, ferner umfassend eine Nasenkanüle, die mit dem Abgaberohr (206, 406) gekoppelt und konfiguriert ist, um das erwärmte und befeuchtete Atemgas in die Nasenlöcher eines Patienten zu leiten.

11. System nach einem der vorstehenden Ansprüche, wobei die Dampfübertragungspatrone Folgendes umfasst:
eine Vielzahl von Hohlfasern;
eine plissierte Membran; oder
eine flache Folienmembran.

12. System nach einem der vorstehenden Ansprüche, wobei die Dampfübertragungspatrone einen beheizten Docht umfasst.

13. System nach einem der vorstehenden Ansprüche, wobei:
das Gaszufuhrmodul ein Gebläse umfasst, das konfiguriert ist, um den Gasstrom des Atemgases auszugeben; und/oder
das System (200, 300) ferner Folgendes umfasst: eine Steuerung, die operativ mit dem Gaszufuhrmodul und dem Flüssigkeitszufuhrmodul gekoppelt und konfiguriert ist, um mindestens einen von einem Gastemperatursollwert, einem Gasdurchsatzsollwert, einem Flüssigkeitsdurchsatzsollwert oder einem Flüssigkeitstemperatursollwert einzustellen.

14. System (200, 300) nach einem der vorstehenden Ansprüche, wobei:
der zweite Flüssigkeitsdurchsatz kleiner ist als der erste Flüssigkeitsdurchsatz und die zweite Luftfeuchtigkeit größer ist als die erste Luftfeuchtigkeit; oder
der zweite Flüssigkeitsdurchsatz größer ist als der erste Flüssigkeitsdurchsatz und die zweite Luftfeuchtigkeit kleiner ist als die erste Luftfeuchtigkeit.

15. System (200, 300) nach einem der vorstehenden Ansprüche, wobei mindestens eines von Folgendem gilt:
die erste Gastemperatur ist 30 bis 40 °C;
der erste Gasdurchfluss ist 5 bis 45 l/min;
mindestens eine der ersten oder zweiten Luftfeuchtigkeit ist 25-45 mg/l.

## Revendications

1. Système de thérapie respiratoire (200, 300) comprenant :
un module d'alimentation en gaz (202, 302) configuré pour fournir un flux de gaz respiratoire ;
un module d'alimentation en liquide configuré pour fournir un flux de liquide, le module d'alimentation en liquide comprenant :
un élément chauffant (327, 427) configuré pour chauffer le liquide ; et
une pompe (224, 324, 424) configurée pour faire circuler le liquide ; et
un module de transfert de vapeur configuré pour recevoir le flux de gaz respiratoire et de liquide, humidifier le gaz respiratoire et évacuer le gaz respiratoire chauffé et humidifié, dans lequel le module de transfert de vapeur comprend une cartouche de transfert de vapeur (216, 416) et un tube de distribution (206, 406), la cartouche de transfert de vapeur (216, 416) comprenant :
une entrée de gaz (464)
configurée pour recevoir le flux de gaz respiratoire provenant du module d'alimentation en gaz ;
une sortie de gaz (465) configurée pour évacuer le gaz respiratoire chauffé et humidifié ;
une entrée de liquide (466) ;
et
une sortie de liquide (467) ;
dans lequel l'entrée de gaz (464) et la sortie de liquide (467) sont disposées à une première extrémité de la cartouche de transfert de vapeur (216, 416), et la sortie de gaz (465) et l'entrée de liquide (466) sont disposées à une seconde extrémité de la cartouche de transfert de vapeur (216, 416), la seconde extrémité de la cartouche de transfert de vapeur (216, 416) étant opposée à la première extrémité de la cartouche de transfert de vapeur (216, 416) de sorte que le liquide et le gaz respiratoire circulent à travers la cartouche de transfert de vapeur (216) dans des directions opposées ;
le système (200, 300) étant configuré pour :
fournir le flux de gaz respiratoire depuis le module d'alimentation en gaz vers le module de transfert de vapeur ;
fournir, pour chauffer le flux de gaz respiratoire, le flux de liquide du module d'alimentation en liquide au module de transfert de vapeur à un premier débit de liquide ;
sortir, à partir de la sortie de gaz (465), le gaz respiratoire chauffé et humidifié du module de transfert de vapeur à une première température de gaz, un premier débit de gaz et une première humidité ;
ajuster le premier débit de liquide à un second débit de liquide, la température de liquide à l'entrée de liquide étant maintenue constante avant et après l'ajustement ;
et sortir le gaz chauffé et humidifié du module de transfert de vapeur à environ la première température de gaz, à environ le premier débit de gaz et à une seconde humidité ;
dans lequel une différence entre la première humidité et la seconde humidité est inversement proportionnelle à l'ajustement du premier débit de liquide au second débit de liquide.

2. Système (200, 300) selon la revendication 1, dans lequel le module d'alimentation en liquide comprend un réservoir (332, 432) configuré pour contenir du liquide et possède un orifice de sortie et un orifice de retour, l'orifice de retour étant en communication fluidique avec le module de transfert de vapeur et configuré pour acheminer le liquide du module de transfert de vapeur vers le réservoir (332, 432).

3. Système (200, 300) selon la revendication 2, dans lequel :
la sortie de liquide de la cartouche de transfert de vapeur (216, 416) est configurée pour acheminer le liquide de la cartouche de transfert de vapeur (216, 416) vers le réservoir (332, 432) par le biais de l'orifice de retour.

4. Système (200, 300) selon l'une quelconque des revendications précédentes, dans lequel le tube de distribution (206, 406) comprend :
un lumen à gaz (407) ; et
un lumen de liquide (474, 475) entourant au moins partiellement le lumen à gaz (407), le lumen à gaz (407) étant en communication fluidique avec la sortie de gaz de la cartouche de transfert de vapeur (216, 416) afin de recevoir le gaz respiratoire chauffé et humidifié de la sortie de gaz de la cartouche de transfert de vapeur (216, 416).

5. Système (200, 300) selon la revendication 4, dans lequel le module de transfert de vapeur et le module d'alimentation en liquide définissent une boucle de circulation de liquide, moyennant quoi le liquide s'écoule du réservoir (332, 432), vers l'élément chauffant (327, 427), vers le lumen de liquide (474, 475) du tube de distribution (206, 406), vers l'entrée de liquide de la cartouche de transfert de vapeur (216), à travers la cartouche de transfert de vapeur (216), et vers le réservoir (332) par le biais de l'orifice de retour.

6. Système (200, 300) selon la revendication 4 ou 5, dans lequel :
le tube de distribution (406) comprend :
une première extrémité ; et
une seconde extrémité, dans lequel le lumen à gaz (407) reçoit le gaz respiratoire chauffé et humidifié à la première extrémité du tube de distribution (406) et achemine le gaz chauffé et humidifié vers la seconde extrémité du tube de distribution (406) ; et
le lumen de liquide (474, 475) comprend :
un premier canal (474) configuré pour acheminer le liquide de la première extrémité du tube de distribution (406) à la seconde extrémité du tube de distribution (406) ; et
un second canal (475) en communication fluidique avec le premier canal (474) à la seconde extrémité du tube de distribution (406) et configuré pour acheminer le liquide de la seconde extrémité du tube de distribution (406) à la première extrémité du tube de distribution (406).

7. Système (200, 300) selon la revendication 6, dans lequel le second canal (475) à la première extrémité du tube de distribution (406) est en communication fluidique avec l'entrée de liquide de la cartouche de transfert de vapeur (416), et l'entrée de liquide reçoit du liquide du second canal (475).

8. Système selon la revendication 6, dans lequel le tube de distribution comprend également un premier capteur de température disposé à l'entrée du premier canal et un second capteur de température disposé à la sortie du second canal.

9. Système selon la revendication 8, dans lequel le premier capteur de température et le second capteur de température mesurent chacun une température, et la différence de température entre les températures mesurées est inférieure à 5 % de la première température de gaz.

10. Système (200, 300) selon l'une quelconque des revendications précédentes, comprenant également une canule nasale couplée au tube de distribution (206, 406) et configurée pour acheminer le gaz respiratoire chauffé et humidifié vers les narines d'un patient.

11. Système selon l'une quelconque des revendications précédentes, dans lequel la cartouche de transfert de vapeur comprend :
une pluralité de fibres creuses ;
une membrane plissée ; ou
une membrane plane.

12. Système selon l'une quelconque des revendications précédentes, dans lequel la cartouche de transfert de vapeur comprend une mèche chauffée.

13. Système selon l'une quelconque des revendications précédentes, dans lequel :
le module d'alimentation en gaz comprend un ventilateur configuré pour fournir le débit de gaz respiratoire ; et/ou
le système (200, 300) comprend également : un dispositif de commande couplé fonctionnellement au module d'alimentation en gaz et au module d'alimentation en liquide et configuré pour ajuster au moins l'un du point de consigne de température de gaz, d'un point de consigne de débit de gaz, d'un point de consigne de débit de liquide ou d'un point de consigne de température de liquide.

14. Système (200, 300) selon l'une quelconque des revendications précédentes, dans lequel :
le second débit de liquide est inférieur au premier débit de liquide, et la seconde humidité est supérieure à la première humidité ; ou
le second débit de liquide est supérieur au premier débit de liquide, et la seconde humidité est inférieure à la première humidité.

15. Système (200, 300) selon l'une quelconque des revendications précédentes, dans lequel au moins l'un :
de la première température de gaz est de 30 à 40 °C ;
du premier débit de gaz est de 5 à 45 L/min ;
d'au moins l'une de la première humidité ou de la seconde humidité est comprise entre 25 et 45 mg/L.
